# EUROPEAN PATENT APPLICATION

(11) **EP 3 195 801 A1**
(43) Date of publication of application: **26.07.2017**
(21) Application number: 15842585.0
(22) Date of filing: 18.09.2015
(51) Int. Cl.: A61B 5/15, A61B 5/154

(54) **ONE-TOUCH DEVICE FOR COLLECTING FLUID**

(30) Priority: 19.09.2014 KR 20140125002
(71) Applicant: JUVIC INC., Seoul 03722 (KR)
(72) Inventor: JUNG, Hyung Il, Seoul 03447 (KR); LI, Chengguo, Suwon-si Gyeonggi-do 16265 (KR)
(74) Representative: Ferroni, Filippo
(86) International application number: PCT/KR2015/009831
(87) International publication number: WO 2016/043554

(57) **Abstract**

The present invention relates to a device for collecting body fluids including (a) a fluid receiving part including a fluid receiving space in which an internal low pressure less than an external atmospheric pressure is formed; (b) a fluid collection part including a hollow microstructure connected to form an open system with the fluid receiving space of the fluid receiving part; and (c) a microstructure hollow barrier for blocking the hollow of the microstructure to maintain the internal low pressure formed in the fluid receiving means.

## Description

### TECHNICAL FIELD

The present invention is supported by the Ministry of Education, Science and Technology (MEST), Republic of Korea (No. 2013054318). The specialized agency of R&D management with regard to the grant number is the National Research Foundation of Korea (NRF), the title of the research program is "Original Technology Research Program", the tile of the research project is "Development of Minimally Invasive and Nonimmune Blood Collection Technique Using Microneedle", the lead agency is the Industry-Academic Cooperation Foundation of Yonsei University, and the project period is from August 16, 2010 to July 30, 2015.

The present patent application claims priority to Korean Patent Application No. 10-2014-0125002 filed with the Korea Intellectual Property Office on September 19, 2014, and the disclosure of the patent application is incorporated by reference herein.

The present invention relates to a one-touch device for collecting fluids.

### BACKGROUND ART

Recently, lab-on-a-chip microfluidic systems and micro-total analysis systems (µ-TAS) have attracted attention as rapid and sensitive methods for point-of-care testing (POCT) [1-4]. A human whole blood sample, an important body fluid, can provide a wide range of useful biological information for diagnosis of various diseases [5]. To obtain blood samples, a number of microsystems have been developed by fusion of actuators and microneedles manufactured by micro-manufacturing technologies such as micro-electro-mechanical systems (MEMS) [6-9].

Numerous solid and hollow microneedles have been designed and manufactured to create micro-scale pathways to the skin for blood sampling, providing minimally invasive and painless methods [10-14]. In addition, micro-actuators have also played an important role in the microsystems by providing power sources for blood extraction [15, 16]. While there are many types of actuation methods, such as electrostatic [17], piezoelectric [18, 19] and magnetic [20] methods, with different designs and operating methods, the shared characteristic of most reported systems is that external power sources (usually heaters or batteries) are required. This limits the miniaturization of the systems, resulting in bulky structure formation. On the other hand, complex manufacturing methods and operating procedures using pressure gradients [21, 22] that do not require additional energy supply and compressed air forces [23] by actuators are not suitable for implementation of real-time analysis systems.

To be a useful tool in a point-of-care testing (POCT) system, a miniature blood extraction system should be devised to reduce the complexity of an actuator structure by reducing the number of external support devices (e.g., heaters or batteries) and functional configurations (holes or valves) while simplifying operating procedures. Thus, the miniature blood extraction system may minimize a system size, lower manufacturing costs, and provide user convenience.

Polydimethylsiloxane (PDMS) has become one of the most popular biocompatible materials in manufacturing microfluidic devices due to attractive features, such as transparency, flexibility, gas penetration, and ease of manufacture using soft lithography [24]. In addition, the surface properties of PDMS may be easily changed by plasma processing, and functional microstructures based on various PDMSs have been developed by utilizing these properties for micro-total analysis systems [25, 26].

Numerous papers and patent documents are referenced and cited throughout this specification. The disclosures of the cited papers and patent documents are incorporated herein by reference in their entirety to clearly convey the level of skill in the art to which the present invention pertains and the content of the present invention.

### TECHNICAL PROBLEM

The present inventors have made extensive efforts to develop a fluid collection device capable of easily collecting body fluids from a subject. As a result, the present inventors have developed a device capable of easily collecting fluids by an operation of applying a force once in a direction perpendicular to a contact surface after being brought into close contact with the skin of a subject, thereby completing the present invention.

Therefore, it is an objective of the present invention to provide a device for collecting body fluids.

It is another objective of the present invention to provide an integrated analysis system for body fluids including the device for collecting body fluids.

Other objectives and advantages of the present invention will become more apparent from the following detailed description of the invention, claims and drawings.

### TECHNICAL SOLUTION

One aspect of the present invention provides a device for collecting body fluids, including:
(a) a fluid receiving part including a fluid receiving space in which an internal low pressure less than an external atmospheric pressure is formed;
(b) a fluid collection part including a hollow microstructure openly connected to form an open system with the fluid receiving space of the fluid receiving part; and
(c) a microstructure hollow barrier for blocking the hollow of the microstructure to maintain the internal low pressure formed in the fluid receiving means. The present inventors have made extensive efforts to develop a fluid collection device capable of easily collecting body fluids from a subject. As a result, the present inventors have developed a device capable of easily collecting fluids by an operation of applying a force once in a direction perpendicular to a contact surface after being brought into close contact with the skin of a subject.

Body fluids collected by the device of the present invention may include various fluids such as blood, interstitial fluid and ocular fluid, preferably blood, more preferably human blood.

The device for collecting body fluids according to the present invention is described below with reference to the drawings.

The device for collecting body fluids according to the present invention may include a fluid receiving part 1 in the form of a chamber including a fluid receiving space 12 in which an internal low pressure less than an external atmospheric pressure is formed, a fluid collection part 2 including a hollow microstructure openly connected to form an open system with the fluid receiving space of the fluid receiving part, and a microstructure hollow barrier 3 for blocking the hollow of the microstructure to maintain the internal low pressure formed in the fluid receiving means.

According to the device for collecting body fluids of the present invention, body fluids may be easily collected by a negative pressure previously formed and maintained in the fluid receiving part. Thus, when fluids are collected, a separate operation of forming a negative pressure in the fluid receiving part is not required. In addition, the device of the present invention is characterized in that a separate process of removing the hollow barrier 3 for maintaining a negative pressure formed in the fluid receiving part is not necessary.

According to the present invention, an internal low pressure less than an external atmospheric pressure formed in the fluid receiving space of the fluid receiving part is also referred to as "negative pressure" herein, and includes a substantially vacuum state. In the present invention, when the fluid receiving part 1, the fluid collection part 2 and the hollow barrier 3 are all combined, the device for collecting body fluids forms a closed system that is not connected to the outside. Therefore, in processes of manufacturing the fluid receiving part 1, the fluid collection part 2, and the hollow barrier 3 and combining the same, a negative pressure may be formed in the fluid receiving space of the fluid receiving part by performing at least the last combining process of finally forming a closed system in a chamber in which a predetermined negative pressure is formed.

As materials used to manufacture the fluid receiving part of the present invention, under an external environment in which a pressure higher than a negative pressure inside the fluid receiving part is formed, any material may be used without limitation, except for materials which are incapable of withstanding a pressure difference between the inside and the outside of the fluid receiving part and materials which lose a restoring force for the original form of a fluid receiving part outer wall 11, thereby causing permanent deformation of the original form and consequently being incapable of maintaining the internal pressure of the fluid receiving part. Preferably, any material known in the art, which is capable of retaining a pre-formed internal negative pressure and is adapted to receive and store collected fluids, may be used. Preferably, to prevent a force acting inside due to a pressure difference with the outside from concentrating on the hollow barrier, elastic materials may be used. Elastomers that may be used in the present invention are specifically, for example, silicone elastomers and polymers formed from precursors such as chlorosilanes (e.g., methylchlorosilane, ethylchlorosilane, and phenylchlorosilane). In particular, a preferred silicone polymer is polydimethylsiloxane (PDMS). Exemplary polydimethyl siloxane polymers are sold under the Sylgard trademark by Dow Chemical Co., Ltd., and in particular, Sylgard 182, Sylgard 184 and Sylgard 186 are suitable. However, when an air-permeable material such as PDMS is used, since it is impossible to maintain an internal negative pressure for a long period of time, the fluid receiving part may preferably be subjected to an airproof treatment.

According to one embodiment of the present invention, the fluid receiving part 1 may include (a) a porous structure having air permeability and (b) a coating agent for coating the exterior of the porous structure such that the porous structure has airproof capability. As a method of forming a negative pressure in the fluid receiving space 12 of the fluid receiving part of the present invention, the following method may be used in addition to the above-described method. The fluid receiving part outer wall 11 is manufactured using a porous material having air permeability, and after manufacturing a device in which the fluid receiving part 1, the fluid collection part 2 and the hollow barrier 3 are all combined, the device is subjected to a coating treatment in a chamber, in which a predetermined negative pressure is formed, to provide airproof capability to the porous material of the fluid receiving part. In this way, a negative pressure may be created in the fluid receiving space 12 of the fluid receiving part. When the porous material of the fluid collecting device permeates the gas at a considerably low rate and a negative pressure inside the device does not substantially change during the time required to complete an airproof treatment, it is also possible to perform an airproof treatment after transferring the device having a negative pressure in a negative pressure chamber to the outside of the chamber.

In the present method, porous materials having air permeability may be used without limitation for manufacturing the fluid receiving part outer wall 11, and specifically, for example, substances having a high gas permeable structure such as polydimethylsiloxane (PDMS) may be used. In the case of the exemplary PDMS, since a gas permeation rate is remarkably low as described above, after a negative pressure is formed in a negative pressure chamber, an airproof treatment may be performed not only inside the chamber but also outside the chamber, thereby facilitating a manufacturing process. In the present method, a material used in a coating treatment, which provides airproof capability to the porous structure of the fluid receiving part outer wall 11, should have a low permeability to gas and moisture, and preferably, materials known in the art as coating materials that enhance biomedical suitability may be used. Specifically, the coating materials may include, for example, parylene, polyzene-F, phosphorylcholine (PC), polylactide, polyglycolide, or a copolymer of polylactide and polyglycolide. All treatment processes, such as a vacuum formation and an airproof treatment, may be carried out with a process of depositing the coating materials described above.

The fluid receiving part 1 of the present invention may have various shapes. It is not necessarily limited to a rectangular parallelepiped or a cubic shape as shown in an embodiment of the present invention. The fluid receiving part may be manufactured in any form such as cylindrical, conical, spherical, hemispherical, pyramidal, and diamond shapes as long as a fluid receiving space capable of forming a negative pressure therein is provided, and such forms do not cause problems for the fluid receiving part to exert the effect of the present invention. In addition, the fluid receiving space 12 of the fluid receiving part is not necessarily limited to a cylindrical shape as shown in an embodiment of the present invention, and may be manufactured in various forms.

The fluid collection part 2 of the present invention may include a hollow microstructure 21 and a support 22 for supporting the hollow microstructure 21. The hollow microstructure 21 alone or in combination with the support 22 is openly connected to form an open system with the fluid receiving part 1. The support 22 of the present invention is a means for assisting the coupling between the fluid receiving part and the microstructure, and need not be manufactured separately from the microstructure. Depending on manufacturing methods of the microstructure, the end portion of the microstructure may be made into the shape of a support, that is, the support may be formed integrally.

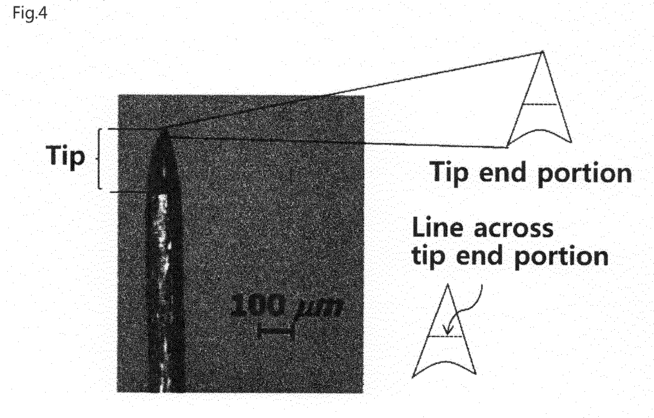

The microstructure constituting the fluid collection part of the present invention is preferably a minimally invasive hollow microstructure for collecting blood developed by the present inventors and disclosed in Korean Patent Application No. 2011-0078510. A detailed description of the hollow microstructure used in the present invention may be explained with reference to the disclosure of Korean Patent Application No. 2011-0078510. In this specification, the term "tip" used in reference to the hollow microstructure refers to the tip of the upper end of the microstructure to which a bevel angle is imparted, and the term "tip portion" refers to a portion from the upper end of the hollow to the end portion of the microstructure and may be observed from the outside by giving a bevel angle to the tip of the upper end of the microstructure (see: FIG. 4).

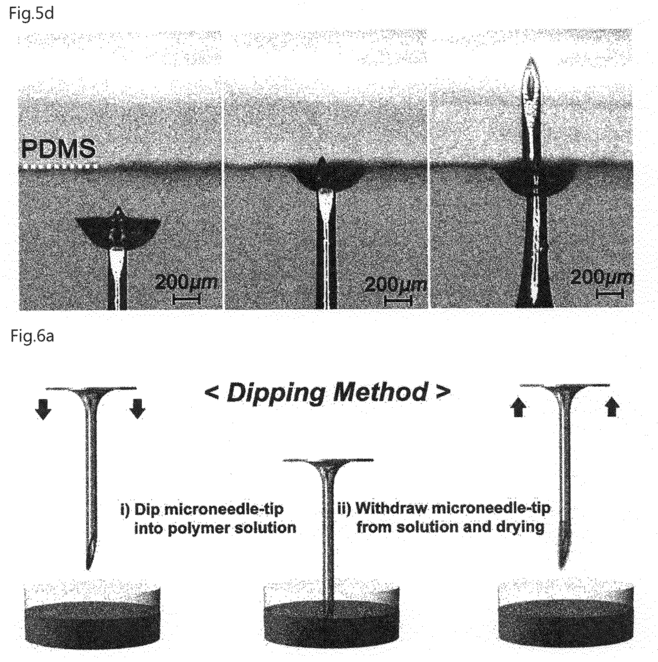

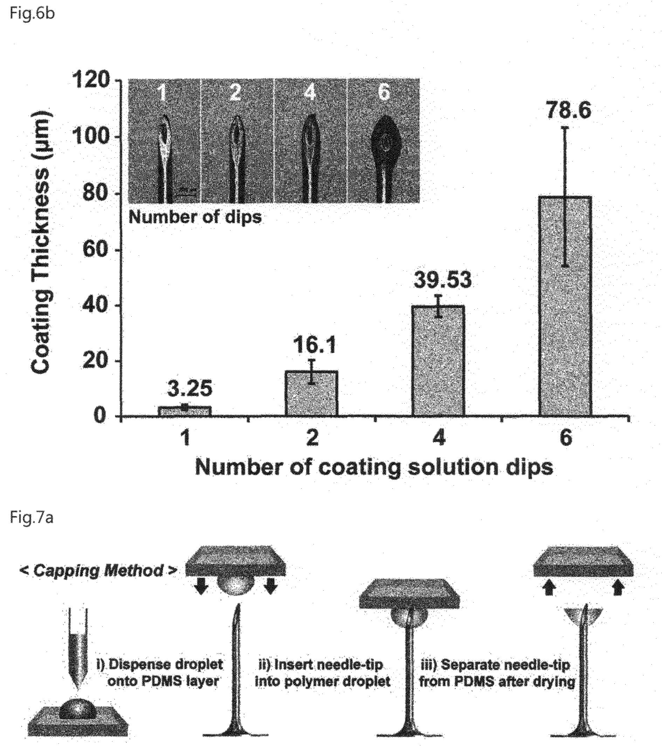

According to one embodiment of the present invention, the microstructure hollow barrier 3 may be a polymer coating 31 formed by a dipping method (see: FIG. 6). The term "dipping method" used in reference to the polymer coating 31, which is the microstructure hollow barrier of the present invention, refers to a method of forming a polymer coating at a desired position by a process of immersing a microstructure as much as a site, where a coating is to be formed, in a viscous substance, and recovering and drying the microstructure (see: FIG. 6a). Since the dipping method according to the present invention is used to form the polymer coating 31 for blocking a hollow at the tip portion of the hollow microstructure, the microstructure may be immersed to a height, at which a hollow at the tip portion of the microstructure is all immersed, and recovered to form the desired polymer coating. In addition, a series of processes in which a microstructure is immersed, recovered and dried are defined as a single dipping process, and the thickness of a polymer coating may be adjusted by controlling the number of dipping processes. For example, increasing the number of the dipping processes increases the thickness of a polymer coating (see: FIG. 6b).

According to one embodiment of the present invention, the polymer coating 31 of the present invention may be composed of a biocompatible and biodegradable viscous material. The biocompatible and biodegradable viscous material of the present invention include substances that are substantially non-toxic to the human body, chemically inert, non-immunogenic, and capable of being degraded by body fluids or microorganisms *in vivo.* In the present invention, viscous materials that are substantially non-toxic to the human body, chemically inert, non-immunogenic, and capable of being degraded by body fluids or microorganisms *in vivo* may be used to form the polymer coating 31 without any particular limitation. As a particular example, the biocompatible biodegradable viscous material is polyvinylpyrolidone (PVP), carboxymethyl cellulose (CMC), hyaluronic acid (HA), polyester, polyhydroxyalkanoates (PHAs), poly(α-hydroxyacid), poly(β-hydroxyacid), poly(3-hydroxybutyrate-co-valerate (PHBV), poly(3-hydroxyproprionate (PHP), poly(3-hydroxyhexanoate (PHH), poly(4-hydroxyacid), poly(4-hydroxybutyrate), poly(4-hydroxyvalerate), poly(4-hydroxyhexanoate), poly(ester amide), polycaprolactone, polylactide, polyglycolide, poly(lactide-co-glycolide) (PLGA), polydioxanone, polyorthoesters, polyetherester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphagens, PHA-PEG, an ethylene vinyl alcohol copolymer (EVOH), polyurethane, silicone, polyester, polyolefin, a polyisobutylene and ethylene-αolefin copolymer, a styrene-isobutylene-styrene triblock copolymer, an acryl polymer and copolymer, a vinyl halide polymer and copolymer, polyvinyl chloride, polyvinyl ether, polyvinyl methyl ether, polyvinylidene halide, polyvinylidene fluoride, polyvinylidene chloride, polyfluoroalkene, polyperfluoroalkene, polyacrylonitrile, polyvinyl ketone, polyvinyl aromatics, polystyrene, polyvinyl ester, polyvinyl acetate, an ethylene-methyl methacrylate copolymer, an acrylonitrile-styrene copolymer, an ABS resin and ethylene-vinyl acetate copolymer, polyamide, alkyd resin, polyoxymethylene, polyimide, polyether, polyacrylate, polymethacrylate, polyacrylic acid-co-maleic acid, chitosan, dextran, celluloses, heparin, alginate, inulin, starch, or glycogen. The biocompatible biodegradable viscous material of the present invention may further include a thickener generally used in the art to control the viscosity thereof The viscosity of the biocompatible biodegradable viscous material may be controlled by adding, as a particular example, hyaluronic acid and a salt thereof, polyvinylpyrrolidone, cellulose polymer, dextran, gelatin, glycerin, polyethyleneglycol, polysorbate, propyleneglycol, povidone, carbomer, gum ghatti, guar gum, glucomannan, glucosamine, dammar gum, rennet casein, locust bean gum, microfibrillated cellulose, psyllium seed gum, xanthan gum, arabino galactan, arabic gum, alginic acid, gelatin, gellan gum, carrageenan, karaya gum, curdlan, chitosan, chitin, tara gum, tamarind gum, tragacanth gum, furcelleran, pectin, or pullulan.

When the dipping process is performed, the viscous material may be dissolved in a solvent to facilitate dipping and withdrawing procedures of the microstructure. The solvent, which is not specifically limited, may be, as a particular example, water, C₁ to C₄ anhydrous or hydrous lower alcohol, acetone, ethyl acetate, chloroform, 1,3-butylene glycol, nucleic acid, diethylether, or butyl acetate, preferably water or lower alcohol, most preferably water.

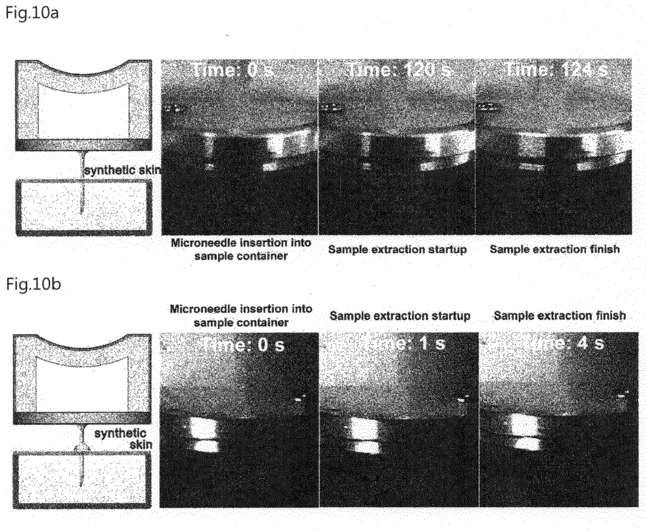

When the skin of a subject is pierced with the fluid collection part 2 to collect fluids using the device for collecting body fluids of the present invention, the polymer coating 31 of the present invention is inserted into the body through the skin barrier with the fluid collection part. Thereafter, the polymer coating undergoes a biodegradation process by fluids or microorganisms in the body, and the closed hollow of the fluid collection part 2 is opened. At this time, due to a negative pressure previously formed in the fluid receiving part 1, fluids naturally move toward the fluid receiving part 1 along the hollow of the fluid collection part 2 (see: FIG. 10a).

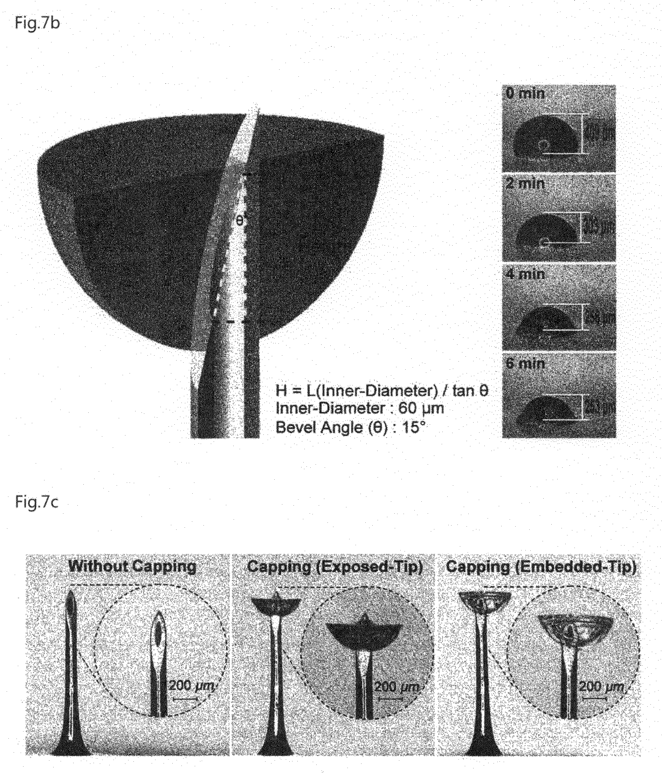

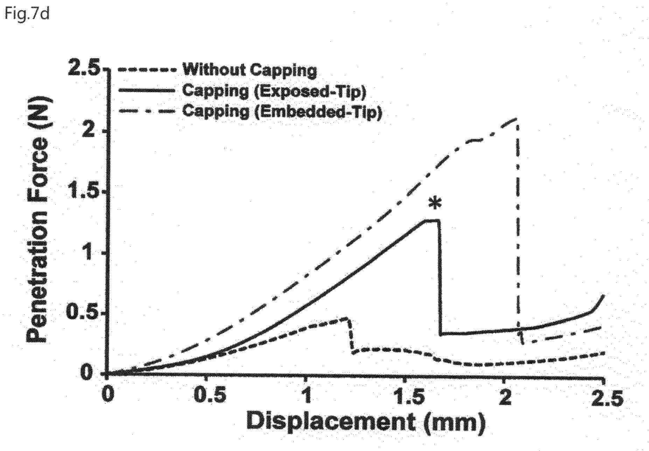

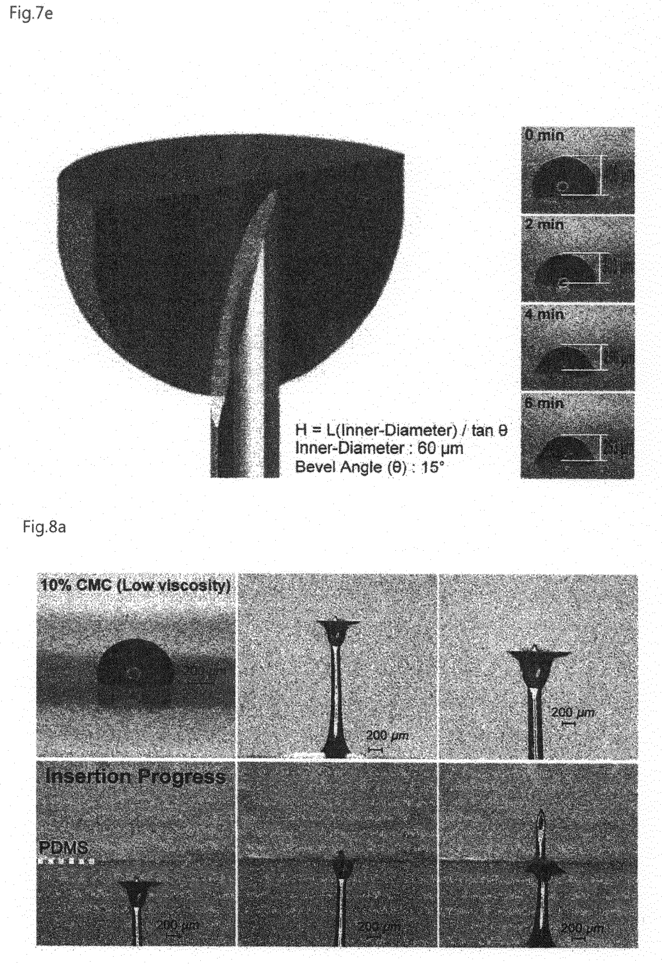

In another embodiment of the present invention, the microstructure hollow barrier of the present invention is a polymer cap 32 formed by a microstructure capping method (see: FIG. 7). The term "capping method" used in reference to the polymer cap 32, which is the microstructure hollow barrier of the present invention, refers to a method of forming the polymer cap 32 by the following processes: (a) polymer droplets are formed on a base layer using a viscous composition, (b) the microstructure is moved in a direction perpendicular to the contact surface between the formed polymer droplets and the base layer so that the microstructure penetrates the formed polymer droplets, and the hollow of the microstructure tip is completely inserted into the polymer droplets so that the tip is not exposed to the outside, and (c) the polymer droplets are dried (see: FIG. 7a). According to one embodiment of the present invention, when body fluids are collected from a subject, the polymer cap 32 may be separated from a microstructure-tip to open a hollow and form a pressure gradient when a vertical force is applied to cause the microstructure to penetrate the skin barrier of the subject. In the case of a device for collecting fluids including the polymer cap 32, fluids may be collected immediately after the fluid collection part 2 is inserted into the body without the hassle of waiting until the hollow barrier 3 is dissolved in the body.

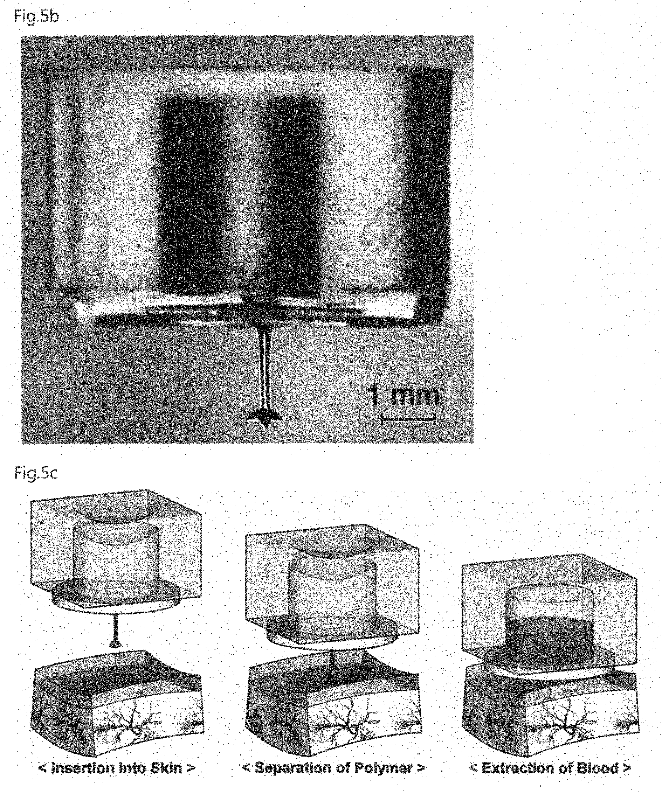

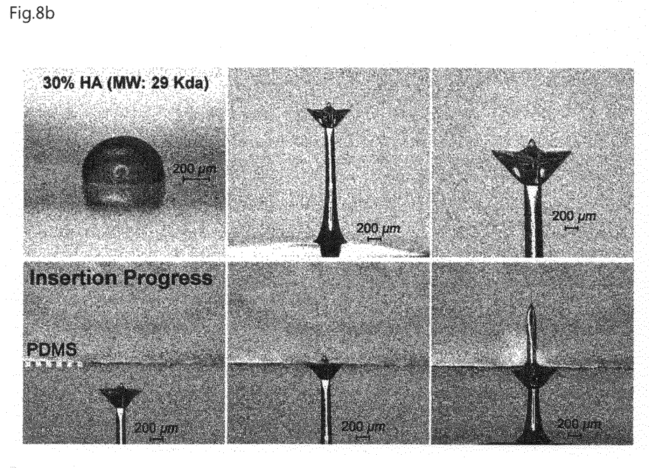

According to one embodiment of the present invention, the polymer cap 32 may form an exposed-tip capping structure (see: FIG. 7b). The exposed-tip capping structure requires a smaller penetrating force when penetrating a skin barrier (see: Table 1) compared to the embedded-tip capping structure (see: FIG. 7e). In addition, in the case of the embedded-tip capping structure, the tip portion of a microstructure does not protrude out of a polymer cap, whereas, in the case of the exposed-tip capping structure, the tip portion of a microstructure protrudes out of a polymer cap. Thus, since the protruding tip portion may be visually confirmed, the microstructure of the exposed-tip capping structure may be inserted more precisely into the desired fluid collection site. According to one embodiment of the present invention, when body fluids are collected from a subject, the polymer cap 32 may be separated from a microstructure-tip and may not enter the skin barrier of the subject. Unlike the polymer coating 31, the polymer cap 32 is not inserted into the body of a subject, and is moved in the opposite direction of the vertical force along the outer wall of a microstructure at the same time as the hollow of the fluid collection part 2 is opened (see: FIGS. 5b and 8). Thus, the polymer cap 32 may be manufactured using a wider variety of materials than the polymer coating 31 inserted into the body. A viscous composition used in manufacturing the polymer cap 32 may be selected from polymers which have a viscosity such that hemispherical polymer droplets may be formed on a base layer and which are capable of forming elastic or inelastic solid forms through a drying process. Specific exemplary materials that may be used to manufacture the polymer coating 31 may be preferably used.

According to one embodiment of the present invention, the fluid receiving part may have transparency. By manufacturing the fluid receiving part to have transparency, the collected fluid may be visually confirmed. Specifically, for example, polydimethylsiloxane (PDMS) having transparency may be used to manufacture a chamber containing a fluid receiving space.

Another aspect of the present invention provides an integrated analysis system for body fluids including the device for collecting body fluids described above in the present invention and a device for analyzing collected fluids. The term "analysis device" used herein refers to any device known in the art for practicing all previously known analytical methods for obtaining information from various fluids collected from the body. A unified analysis system may be constructed by integrating the device for collecting body fluids of the present invention and the analysis device. The device for collecting fluids of the present invention and the analysis device may be openly connected with each other or may form a closed system respectively by at least one blocking means. The blocking means may be opened, if necessary, to form a passage through which fluids may move between the fluid receiving part of the device for collecting fluids and the analysis device.

According to one embodiment of the present invention, the device for analyzing collected fluids may include a signal device for generating a fluid analysis signal. The signal device of the analysis device used in the integrated analysis system of the present invention may include probes with a microchannel or microarrays with antibodies (or microchips), biosensors, immunochromatography-based analysis devices (e.g., rapid kits), ELISA kits, polymerase chain reaction (PCR) analysis devices and real-time PCR analysis devices, without being limited thereto.

### ADVANTAGEOUS EFFECTS

The features and advantages of the present invention are summarized as follows: (a) the present invention provides a device for collecting body fluids. (b) the present invention provides an integrated analysis system for body fluids including the device for collecting body fluids. (c) the device of the present invention is easy to manufacture, can easily collect body fluids, can be transported, and can efficiently collect fluids. (d) the device for collecting fluids of the present invention can be integrated with an analysis device to perform collection and analysis of fluids at the same time.

### DESCRIPTION OF DRAWINGS

FIG. 1 illustrates one embodiment of a device for collecting body fluids. 1: FLUID RECEIVING PART; 2: FLUID COLLECTION PART; 3: HOLLOW BARRIER; 11: FLUID RECEIVING PART OUTER WALL; 12: FLUID RECEIVING SPACE; 21: HOLLOW MICROSTRUCTURE; 22: SUPPORT.

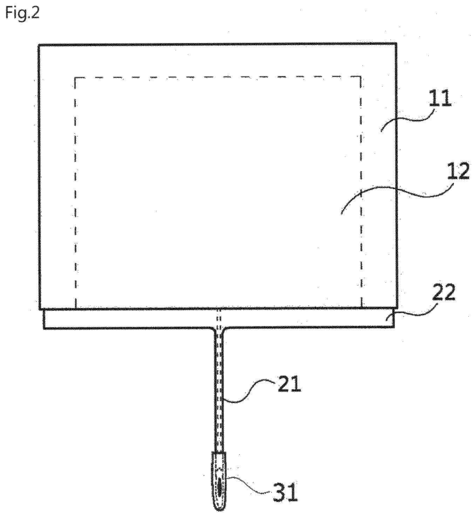

FIG. 2 illustrates one embodiment of a device for collecting body fluids including a polymer coating which is a hollow barrier formed by a dipping method. 31: POLYMER COATING.

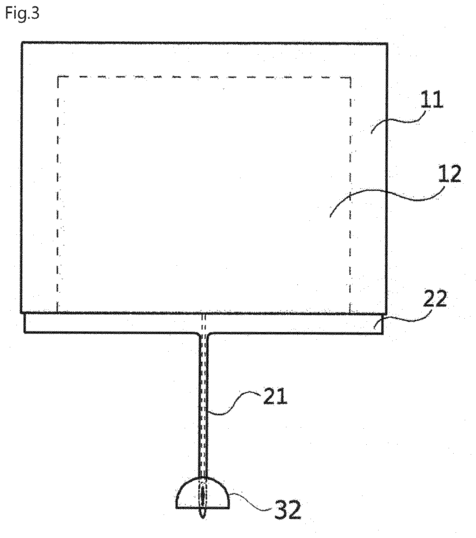

FIG. 3 illustrates one embodiment of a device for collecting body fluids including a polymer cap which is a hollow barrier formed by a capping method. 32: POLYMER CAP.
FIG. 4 illustrates one embodiment of the distal end of a hollow microstructure.
FIG. 5 is a schematic diagram showing one embodiment of a device for collecting body fluids. FIG. 5a is an exploded view showing each part of a device for collecting body fluids, which includes a vacuum PDMS chamber openly connected with a hollow microstructure having a polymer cap. FIG. 5b shows an embodiment of a device for collecting body fluids. FIG. 5c shows one embodiment showing the principle of blood collection using a device for collecting body fluids. FIG. 5d shows a state in which a polymer cap formed on the tip of a microstructure is removed.
FIG. 6 illustrates formation of a hollow barrier by a dipping method. FIG. 6a shows the procedure of the dipping method for forming a polymer coating, and FIG. 6b is a graph showing an increase in the thickness of a polymer coating according to the number of a dipping process.
FIG. 7 illustrates formation of a hollow barrier by a capping method. FIG. 7a shows the procedure of the capping method for forming a polymer cap, FIG. 7b is a schematic view of an exposed-tip capping structure, and FIG. 7c shows one embodiment of the exposed-tip capping structure and an embedded-tip capping structure, which are formed by the capping method. FIG. 7d shows a result of measuring penetration force of a device in which a polymer cap having an exposed-tip or embedded-tip capping structure is formed. FIG. 7e is a schematic view of the embedded-tip capping structure.
FIG. 8 shows embodiments of polymer caps. FIG. 8a shows embodiments of polymer caps manufactured using carboxymethyl cellulose (CMC) and the uses thereof, and FIG. 8b shows embodiments of polymer caps manufactured using hyaluronic acid (HA) and the uses thereof.
FIG. 9 is a schematic diagram showing a pre-vacuum activation and a parylene film deposition process of a device for collecting body fluids. FIG. 9a is a cross-sectional view illustrating vacuum and airproof treatments of a device for collecting body fluids.
FIG. 9b shows the fluid receiving part outer wall before parylene coating, and FIG. 9c shows the fluid receiving part outer wall after parylene coating.
FIG. 10 shows an embodiment of the use of a device for collecting body fluids in a simulated body fluid model. FIG. 10a shows an embodiment of the use of the device in which a hollow is blocked by a polymer coating, and FIG. 10b shows an embodiment of the use of the device in which a hollow is blocked by a polymer cap.
FIG. 11 shows the results of experiments on the relationship between a fluid capacity and the volume of a fluid receiving space.

### MODES OF THE INVENTION

Hereinafter, the present invention will be described in detail by describing exemplary embodiments of the invention. It will be obvious to those skilled in the art that these embodiments are only provided to more particularly explain the present invention and the scope of the present invention is not limited thereto.

### Examples

### Example 1: Manufacture of device for collecting fluids

A painless hollow microstructure with an optimized structure for minimally collecting invasive body fluid was manufactured by a drawing lithography-based technology based on conventional technologies [29, 30]. A polydimethylsiloxane (PDMS) chamber was prepared by pouring a 10:1 (v/v) prepolymer mixture of Sylgard 184 elastomer and a fining agent (manufactured by Dow Corning) onto an aluminum master. The mixture was cured in an 80 ° C oven for one hour, and the resulting PDMS mold was carefully peeled off from the master. Subsequently, the hollow microstructures were assembled in a concentric shaft, thereby completing a one-touch body fluid collection instrument not having a polymer cap. Three aluminum masters were used to manufacture PDMS chambers having different volumes. The internal heights of the cylindrical chambers were 4 mm, and the internal diameters thereof were 3, 4, and 5 mm, respectively. Three PDMS chambers having different volumes of 28.26, 50.24, and 78.50 µl were manufactured.

### Example 2: Manufacture of hollow microstructure, hollow of which is blocked by polymer coating or polymer cap

### 2-1. Polymer solution

Polyvinylpyrrolidone (PVP, 36 kDa, Sigma), carboxymethylcellulose (CMC, 90 kDa, low-viscosity, Sigma), and sodium hyaluronic acid (HA, 29 kDa, Soliance) were used as substrate polymers to block a hollow structure of a tip of a microstructure by dipping and capping methods. A PVP powder was dissolved in each of green and red dye solution (1%, w/v) at room temperature to prepare green and red dyes (Tartrazine, Bowon) containing a viscous PVP (35%, w/v) solution. CMC and HA powders were respectively dissolved in pink and yellow dye solutions (1%, w/v), thereby preparing a pink dye (Tartrazine, Bowon) containing a CMC (10%, w/v) solution and a yellow dye containing a HA(30%, w/v) solution, respectively.

### 2-2. Dipping method

A hollow microstructure was fixed in the downward direction on a micropositioner (Syringe pump, New Era Pump Systems) used to control position and a dipping rate. The microstructure was vertically dipped at a rate of 50 mm/min into a reservoir containing a 100 µl polymer coating solution until a hollow structure of a tip of the microstructure was completely submerged in a viscous PVP (35%, w/v) solution. A speed used to collect microstructure from a coating solution was maintained at 5 mm/min so as to form a polymer film on a surface of the tip of the microstructure. Immediately after the collection step, the coated polymer was air-dried. This dip-coating procedure was repeated until a polymer coating 31 having a desired thickness was obtained.

### 2-3. Capping method

A single polymer droplet was prepared using a solution dispenser (ML-5000X, Musashi) and automated X, Y, and Z stages (SHOT mini 100-s, Musashi) by discharging previously prepared polymer solutions onto the surface of a PDMS base layer at 0.9 kgf/cm for 0.05 seconds. The prepared PDMS base layer with the dispensed polymer droplet was fixed in the downward direction on a micropositioner (syringe pump, New Era Pump Systems) and was in contact with a microneedle, which was placed below the micropositioner, until the sharp tip of the microneedle just passed through the center of the droplet and was inserted into the PDMS base layer at a rate of 5 mm/min at room temperature for 4 min, to solidify the polymer. Finally, an isolation step was performed to separate the microstructure, on which the polymer-capped were formed, from the PDMS base layer at a rate of 50 mm/min.

### 2-4. Mechanical strength analysis

The mechanical force required for a tip-blocked microstructure to penetrate the skin was measured by a displacement-force analyzer (Z0.5TN, Zwick) using 0.4-mm-thick synthetic skin (Gilchrist et al. 2008, Polyurethane elastomers, John Burn Inc., UK), which simulated the high-resistance stratum corneum of human skin. The tip-blocked microstructure was attached to the sensor probe of the force analyzer and pressed against the synthetic skin sheet at a speed of 60 µm/s via penetration force application. The penetration force of the tip-blocked microstructure was determined by measuring the maximum force before the penetration. The force was recorded as a function of displacement associated with the microstructure.

### Example 3: Pre-vacuuming and coating with parylene

The Parylene-C dimer (Femto Science Co.) and the microprocessor-controlled parylene coating system (Femto Science Co.) are commonly used to perform surface treatment with parylene. A parylene-C film with a thickness of 1 µm was thermally deposited on the surface of the closed blood extraction system by the following polymerization steps: (1) evaporation: parylene-C dimers (di-para-xylene) were first vaporized at a temperature of 160 °C, (2) pyrolysis: the dimers were pyrolyzed at a temperature of 650 °C to form a highly reactive monomer (para-xylene) of parylene-C, and (3) deposition: the resulting polymer (poly-para-xylene) was finally coated onto the blood extraction system surface at room temperature. All of these processes were performed under vacuum conditions of less than 0.1 torr for 1.5 hours. The thickness of the parylene-C film was controlled by setting a quartz crystal microbalance (QCM) in the deposition chamber, and the film thickness was calculated by monitoring a frequency change in the QCM during deposition. The QCM change was measured from the beginning of the evaporation step, and the deposition was finished when the QCM frequency shift reached the value corresponding to the target thickness of the parylene-C film.

### Example 4: Characterization of surface properties

The surface morphologies of the vacuum PDMS chamber with parylene coating were characterized by scanning electron microscopy (SEM; JEOL-7001, JEOL Ltd.) at an accelerating voltage of 15 kV. All samples were coated with a thin platinum layer by means of a sputtering machine for 100 seconds to produce a conductive surface prior to observation by SEM.

### Example 5: Operation of device for collecting body fluid

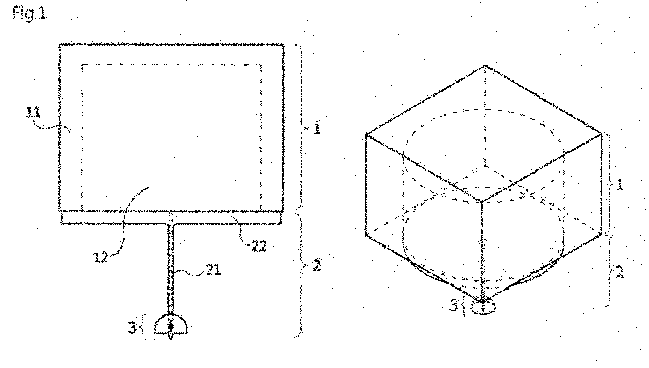

FIG. 1c illustrates an operation principle of an embodiment of the device for collecting body fluids of the present invention.

The top of the microstructure tip of the fluid collection part of the device for collecting body fluids of the present invention was attached to a fluid collection site of a subject and was applied with a vertical force greater than the penetrating force that could penetrate the skin barrier of the subject so that the fluid collection part 2 was inserted into the subject skin.

In the case of a device with the polymer coating 31, the hollow of the fluid collection part was opened after a lapse of a decomposition time of the polymer coating and thus pressure gradients were formed, whereby a fluid from a subject was collected into the fluid receiving space 12 of the fluid receiving part 1.

In the case of the device with the polymer cap 32, the fluid collection part was inserted into the subject skin and, at the same time, the polymer cap 32 was removed. Immediately thereafter, pressure gradients were formed inside and outside the fluid receiving part, and thus, fluid from the subject was collected into the fluid receiving space 12 of the fluid receiving part 1.

### Example 6: In vitro blood extraction

The liquid extraction capability of the blood extraction device was evaluated using distilled water (DW) and human whole-blood samples treated with 8% (v/v) anticoagulant solution CPDA-1. Each system was attached to a pusher, which was connected to the micropositioner (syringe pump, New Era Pump Systems) to allow accurate control of the insertion process for liquid extraction. The device was driven at a rate of 50 mm/min to insert the polymer-capped microstructure into the sample container with a synthetic skin cover to perform one-touch liquid extraction. After sampling, the system was removed from the sample container, and the extraction volumes of the liquid samples were measured using a 100 µl syringe (Hamilton).

### Example 7: Blood extraction amount control according to volume control of fluid receiving space 12 of fluid receiving part 1

A blood collection experiment was performed by means of a device for collecting a fluid equipped with a single hollow microstructure. This experiment was performed using a hollow microstructure with a 15° bevel angle and an inner diameter of 60 µm and a PDMS chamber with an internal negative pressure having a liquid containment space of 28.26 µl, 50.24 µl, or 78.50 µl. Blood collection was performed by applying the one-touch blood collection device to the virtual skin reproduction chamber containing a blood sample.

### Results

### 1. Self-powered one-touch body fluid extraction device

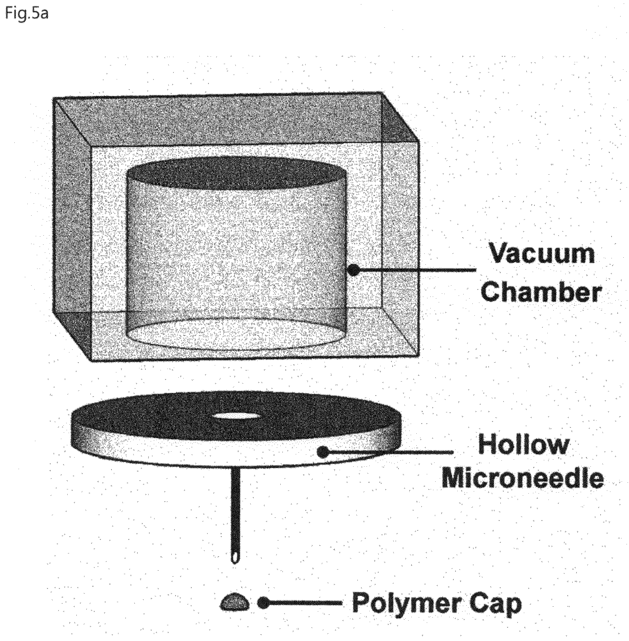

As shown in FIGS. 1 and 5A, the self-powered one-touch body fluid extraction system consists of three parts: 1) a fluid receiving part for providing power for the blood extraction by pressure gradient-driven force and for storing the extracted blood sample; 2) a fluid collection part including a hollow microstructure, which was minimally invasive and optimized for the skin, to induce the micro-channel for blood sampling; and 3) a hollow barrier for selectively blocking the hollow structure of the tip of the microstructure to maintain a closed pre-vacuum system and for precisely controlling the initiation of the blood sampling process.

A PDMS chamber corresponding to a fluid receiving part was manufactured using a traditional microfabrication technique [27, 28] and was designed as a 7 mm × 7 mm × 5 mm cube, with a cylindrical chamber having a 4 mm height and a 4 mm inside diameter. The hollow microstructure with an inner diameter of 60 µm, an outer diameter of 130 µm, and a 15° bevel angle was fabricated by drawing lithography, and the use of this optimized microstructure for minimally invasive blood extraction was demonstrated in previous studies [29, 30]. A real microscopic image of this system is illustrated in FIG. 5b.

The operating principle of this system is illustrated in FIG. 5c. The one-touch press system induces insertion of the hollow microstructure 21 into the skin by inducing the separation of the polymer cap 32 from the tip of the microstructure under the resistance force of the skin barrier. Simultaneously, the blood sample was extracted into the PDMS chamber by the pressure gradient between the fluid receiving space 12 of the fluid receiving part, in which was formed, and the blood vessel. The transparent property of the PDMS layer facilitates observation of a process of separating the polymer cap from the tip of the microstructure, and thus, is was used to reproduce the barrier function of the skin. A functional separation principle of the polymer cap 32 is illustrated in FIG. 5d.

### 2. Tip for blocking hollow microstructure

In particular, biocompatible polymers PVP, CMC, and HA have been widely used as structural materials for the fabrication of coated and dissolved microstructures in medical applications based on micro-fabrication techniques such as a dip-coating method and drawing-lithograph [31, 32]. In the present invention, the optimal concentrations of such polymers were used to manufacture a tip-blocked hollow microstructure structure using dipping and capping methods. Subsequently, to induce pre-vacuum activation, as a subsequent process, for the closed one-touch blood extraction system, the tip-blocked microstructure was openly connected to the PDMS chamber.

### 3. Polymer-coated hollow microstructure

The hollow microstructure was coated with viscous PVP (35%, w/v) solution with red dye using a microscale dipping method developed previously [33, 34]. As shown in FIG. 6a, the dipping method consisted of two distinct steps occurring in sequence: i) a dipping step: controlling to dip the tip of the microstructure into the polymer solution to determine a coating area and ii) a withdrawal and drying step: withdrawing the tip of the microstructure from a polymer solution at a rate of 5 mm/min and coating a viscous PVP polymer on a surface of the microstructure. By the drying, a solid polymer coating was attached onto the tip of the microstructure.

In manufacturing the polymer-coated microstructure, it is important to block the hollow structure of the tip of the microstructure and allow the polymer coating to attach to the tip of the microstructure without destruction during subsequent processes, such as pre-vacuum treatment, for example, by keeping an interior pressure of the vacuum chamber lower.

To accomplish this, microstructures having different polymer coatings were manufactured, connected to vacuum chambers, and subjected to experiments. The thicknesses of the polymer coatings were controlled by the number of dips; polymer coating thicknesses of 3.25 ± 0.44 µm, 16.1 ± 2.09 µm, 39.53 ± 1.93 µm, and 78.6 ± 12.26 µm were obtained by dipping the tip of the microstructure into the coating solution one, two, four, and six times, respectively. Results are illustrated in FIG. 6b. The thickness of the polymer coating on the microneedle tip increased as the number of dips increased. To guarantee physical strength to ensure block of the hollow structure under a lower pressure condition, a polymer coating thickness was determined to be at least 39.53 ± 1.93 µm (dipping into the coating solution four times).

### 4. Polymer-capped hollow microstructure

The hollow microstructure was capped with a viscous PVP (35%, w/v) polymer solution with green dye using a novel micron-scale capping method suggested by the present inventors. As shown in FIG. 7a, this capping method consists of three steps.
(i) First, a capping polymer solution is provided on the surface of a PDMS base layer to form a single polymer droplet. In particular, the height of the solid polymer cap on the tip of the microstructure can be controlled by adjusting the amount of the polymer in a droplet using a dispenser. As illustrated in FIG. 7b, it was required to provide a size higher than 204 µm (H=L (an inner diameter of hollow microstructure)/tan θ =60/tan15°) so as to guarantee complete block of the needle. With increasing drying time of a polymer droplet, the height of the polymer droplet was decreased. However, after four minutes, the height of a solidified structure was not changed because evaporation of distilled water was completed after four minutes. Therefore, to form a solid polymer cap having a height of 250 µm, it is required to provide a polymer droplet having a height of at least 400 µm on a surface of the PDMS base layer. Since a surface of the PDMS base layer is hydrophobic, a contact angle of the PDMS base layer to the polymer solution may be decreased by increasing the height of the droplet, and the polymer solution may be easily isolated along with the solid polymer cap in the isolation step.
(ii) In a second step, a polymer droplet fixed in the downward direction was precisely controlled to contact with the tip of the microstructure. As illustrated in FIG. 7c, an embedded-tip (see FIG. 7e) or exposed-tip (see FIG. 7b) capping structure may be manufactured by controlling the position of the microstructure tip in the droplet. To form the tip of the microstructure with an embedded-tip capping structure, the microstructure tip was inserted into the middle of a polymer droplet without insertion into a PDMS base layer. On the other hand, to form an exposed-tip capping structure, the top of the tip of the microstructure was inserted into a smooth PDMS base layer via a droplet. To visually control the position of the microstructure tip inside the PDMS base layer, the transparent property of PDMS was used. (iii) Finally, to solidify the polymer, the microstructure was allowed to stand at room temperature for four minutes and then a separation process of separating the polymer-capped microstructure from the PDMS based layer was performed.

To accomplish successful blood extraction by means of a microstructure, the microstructure should have strength sufficient to penetrate the skin barrier without damage. Both the blocking microstructures by capping exhibit penetration force smaller than an average fracture force (5.85 ± 0.25 N) of hollow microstructures. Penetration forces are summarized in Table 1.

**Table 1**

| | Penetration force (N) | Displacement (mm) |
|---|---|---|
| No blocking | 0.78 ± 0.01 | 1.47 ± 0.03 |
| Blocking by capping (exposed tip) | 1.31 ± 0.01 | 1.63 ± 0.02 |
| Blocking by capping (embedded tip) | 2.30 ± 0.10 | 2.13 ± 0.05 |

Using a micron-scale capping method, the hollow microstructures were respectively capped with viscous CMC (10%, w/v) and HA (30%, w/v) polymer solutions respectively including pink and yellow dyes. A polymer cap separation process is illustrated in FIG. 8.

### 5. Pre-vacuum activation and parylene film deposition

The high gas permeability properties of PDMS were used to induce inner pre-vacuum activation for the closed system. On the other hand, the gas leaking through the pores of PDMS made it difficult to maintain the applied negative pressure. Parylene is widely used as an inert coating material to enhance biomedical compatibility due to its low permeability to gases and moisture, and may be coated on any geometric surfaces even having holes and cracks [35, 36]. Therefore, to form a highly closed system, the pre-vacuum activation of the one-touch blood extraction system may be maintained over a long period of time (more than 10 hours) by allowing the parylene deposition to effectively seal the porous structure of the PDMS chamber. The contents were confirmed by the Satoshi Konisni group [37]. All of the procedures for vacuumizing and airproofing were performed by a single parylene film deposition process.

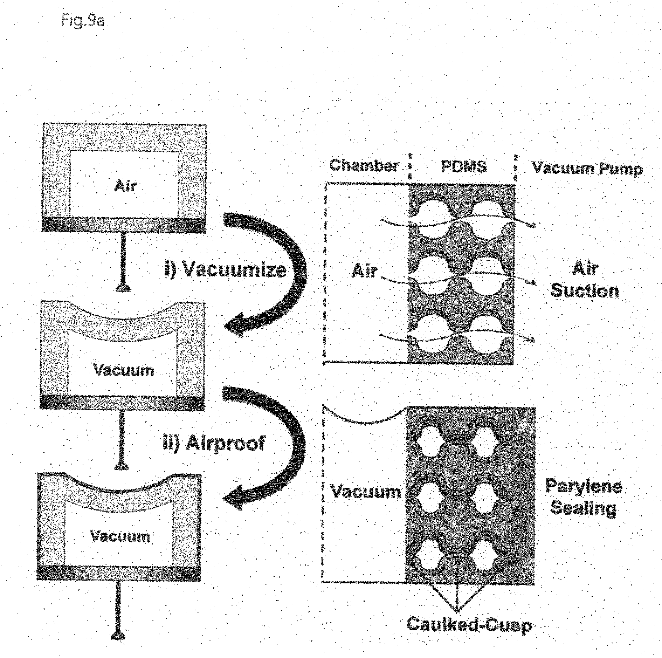

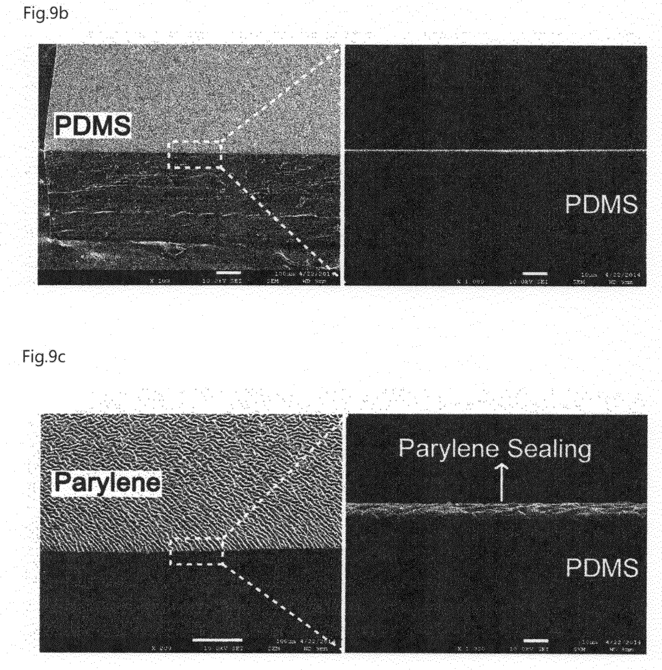

First, all of the closed blood extraction systems were formed by blocking the tip of the microstructure with the polymer using the capping methods. Subsequently, pre-vacuum activation was performed by maintaining a low-pressure condition for at least 20 minutes. The low-pressure condition was established before a parylene film deposition process, and a low vacuum condition of less than 0.1 torr was maintained for 1.5 hours. FIG. 9a schematically illustrates the pre-vacuum activation and parylene film deposition process. Air molecules form vacuum through the porous structure of the PDMS to form a vacuum chamber. Subsequently, parylene was deposited on the surface of PDMS to successfully seal the porous structure with parylene caulking inside and with 1 µm-thick parylene coating outside. FIGS. 9b and c illustrate the scanning electron microscope (SEM) images of the PDMS chamber surface before and after the deposition with parylene and 1 µm-thick parylene film coating. Potential energy was stored inside the pre-vacuum activated blood extraction system which has been airproofed, thereby providing the driving force for blood sampling when interconnected with a blood vessel via the channel of the hollow microneedle. The pre-vacuum activation mechanism demonstrated that this system can be used for blood sampling without the need for an external power source and, further, the volume of an extracted sample may be adjusted by controlling the volume of the vacuum chamber.

### 6. Fluid extraction control

Press force was applied to the blood extraction system to induce insertion of the microstructure into the sample container by passing through the synthetic skin cover for the liquid extract. It was observed that the polymer coated on the tip of the microstructure was inserted into the container with the microstructure without residues on a surface of the synthetic skin by the dipping method. Accordingly, to dissolve the polymer coated on the tip of the microstructure, sample extraction was initiated after 120 seconds of insertion of the microstructure into the sample container and this extraction process was completed within four seconds. However, the capping polymer was removed for the surface of the microstructure tip and moved in a downward direction due to resistance of a synthetic skin. As illustrated in FIG. 10, extraction was immediately performed within four seconds.

### 7. Blood extraction volume control

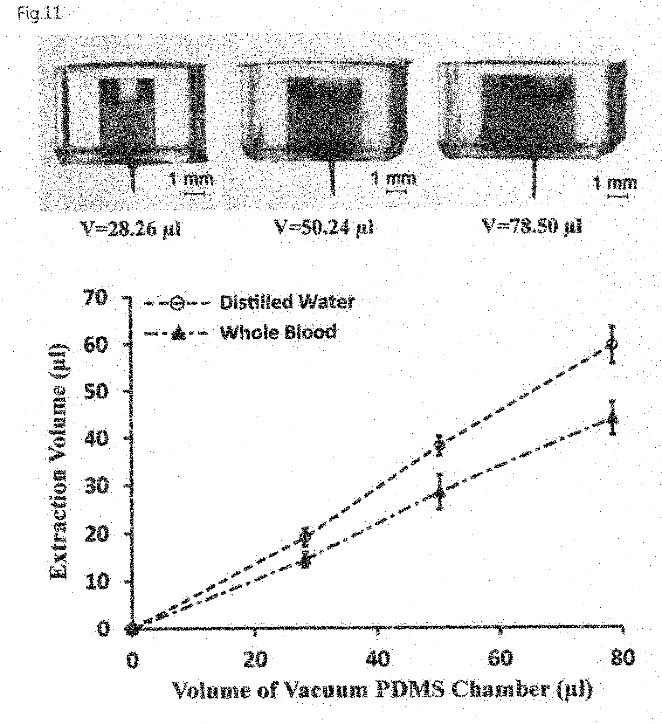

Blood extraction was performed using a device equipped with each of vacuum PDMS chambers having different volumes of 28.26 µl, 50.24 µl, and 78.50 µl. As results, blood samples were respectively obtained in total volumes of 14.3 µl, 28.3 µl, and 43.7 µl sufficient for microsystem analysis within five seconds (see FIG. 11).

Although some embodiments of the present invention have been described in detail, it is obvious to those skilled in the art that the embodiments are merely provided as preferred examples and the scope of the present invention is not limited thereto. Therefore, the substantial range of the present invention is defined by the appended claims and equivalent thereof.

### References

1. V. Linder, Analyst, 2007, 132, 1186-1192.
2. A. Arora, G. Simone, G. B. Salieb-Beugelaar, J. T. Kim and A. Manz, Analytical Chemistry, 2010, 82, 4830-4847.
3. L. Gervais, N. De Rooij and E. Delamarche, Advanced Materials, 2011, 23, H151-H176.
4. A. K. Yetisen, M. S. Akram and C. R. Lowe, Lab on a Chip, 2013, 13, 2210-2251.
5. M. Toner and D. Irimia, Annual Review of Biomedical Engineering, 2005, 7, 77.
6. A. Oki, M. Takai, H. Ogawa, Y. Takamura, T. Fukasawa, J. Kikuchi, Y. Ito, T. Ichiki and Y. Horiike, Javanese Journal of Applied Physics, 2003, 42, 3722.
7. G. E. Gattiker, K. V. Kaler and M. P. Mintchev, Microsystem Technologies, 2005, 12, 44-51.
8. T. Li, A. Barnett, K. L. Rogers and Y. B. Gianchandani, Lab on a Chip, 2009, 9, 3495-3503.
9. A. V. Ganesan, H. Kumar, S. Swaminathan, K. Singh, R. A. Joy, N. Sood, T. Gokhale and R. Mittal, BioNanoScience, 2014, 4, 128- 135.
10. H. J. Gardeniers, R. Luttge, E. J. Berenschot, M. J. DeBoer, S. Y. Yeshurun, M. Hefetz, R. van't Oever and A. van den Berg, Journal of Microelectromechanical Systems, 2003, 12, 855-862.
11. K. Kim, D. S. Park, H. M. Lu, W. Che, K. Kim, J.-B. Lee and C. H. Ahn, Journal of micromechanics and Microengineering, 2004, 14, 597.
12. P. Zhang, C. Dalton and G. A. Jul Hen, Microsystem technologies, 2009, 15, 1073-1082.
13. B. P. Chaudhri, F. Ceyssens, P. De Moor, C. Van Hoof and R. Puers, Journal of Micromechanics and Microengineering, 2010, 20, 064006.
14. K. Tsuchiya, S. Jinnin, H. Yamamoto, Y. Uetsuji and E. Nakamachi, Precision Engineering, 2010, 34, 461-466.
15. A. Nisar, N. Afzul purkar, B. Mahaisavariya and A. Tuantranont, Sensors and Actuators B: Chemical, 2008, 130, 917-942.
16. M. W. Ashraf, S. Tayyaba and N. Afzul purkar, International Journal of Molecular Sciences, 2011, 12, 3648-3704.
17. W.-y. Liu, Research on Electrostatic Micropump Pul 1-in Phenomena Based On Reduced Order Model, 2010.
18. K. Tsuchiya, N. Nakanishi, Y. Uetsuji and E. Nakamachi, Biomedical Microdevices, 2005, 7, 347-353.
19. G. Liu, C. Shen, Z. Yang, X. Cai and H. Zhang, Sensors and Actuators A: Physical, 2010, 163, 291-296.
20. A. Al-Halhouli, M. Kilani and S. Bu, Sensors and Actuators A: Physical, 2010, 162, 172-176.
21. Fujioka, S. Khumpuang, M, Horede and S. Sugiyama, Novel pressure-gradient driven component for blood extraction, 2005.
22. S. Khumpuang, K. Fujioka and S. Sugiyama, Development of bio-chemical sensor system integrated with blood extraction device, 2007.
23. C. G. Li, K, Lee, C, Y, Lee, M. Dangol and H. Jung, Advanced Materials, 2012, 24, 4583-4586.
24. I. K. Dimov, L. Basabe-Desmonts, J. L. Garcia-Cordero, B. M. Ross, A. J. Ricco and L. P. Lee, Lab on a Chip, 2011, 11, 845- 850.
25. G. S. Fiorini and D. T. Chiu, Bio Techniques, 2005, 38, 429-446.
26. A. Z. Li, Y. L. Hou, Y. W. Zhang and L. Zhu, Analytical Methods, 2014.
27. B,-H. Jo, L. M. Van Lerberghe, K. M. Motsegood and D. J. Beebe, Journal of Microelectromechanical Systems, 2000, 9, 76-81.
28. N. L. Jeon, D. T. Chiu, C. J. Wargo, H. Wu, I. S. Choi, J. R. Anderson and G. M. Whitesides, Biomedical Microdevices, 2002, 4, 117-121.
29. K. Lee, H. C. Lee, D. S. Lee and H. Jung, Advanced Materials, 2010, 22, 483-486.
30. C. G. Li, C. Y. Lee, K. Lee and H. Jung, Biomedical Microdevices, 2013, 15,17-25.
31. Y.-C. Kim, J.-H. Park and M. R. Prausnitz, Advanced Drug Delivery Reviews, 2012, 64, 1547-1568.
32. J. D. Kim, M. Kim, H. Yang, K. Lee and H. Jung, Journal of Controlled Release, 2013, 170, 430-436.
33. H. S. Gill and M. R. Prausnitz, Journal of Controlled Release, 2007, 117, 227-237.
34. H. S. Gill and M. R. Prausnitz, Pharmaceutical Research, 2007, 24, 1369-1380.
35. T. Y. Chang, V. G. Yadav, S. De Leo, A. Mohedas, B. Rajal ingam, C.-L. Chen, S. Selvarasah, M. R. Dokmeci and A. Khademhosseini, Langmuir, 2007, 23, 11718-11725.
36. G.-Y. Lee, Y.-H. Choi, H.-W. Chung, H. Ko, S. Cho and J.-C. Pyun, Biosensors and Bioelectronics, 2013, 40, 227-232.
37. S. Sawano, K. Naka, A. Werber, H. Zappe and S. Konishi, Sealing method of PDMS as elastic material for MEMS, 2008.

## Claims

1. A device for collecting body fluids comprising:
(a) a fluid receiving part comprising a fluid receiving space in which an internal low pressure less than an external atmospheric pressure is formed;
(b) a fluid collection part comprising a hollow microstructure connected to form an open system with the fluid receiving space of the fluid receiving part; and
(c) a microstructure hollow barrier for blocking a hollow of the microstructure to maintain the internal low pressure formed in the fluid receiving means.

2. The device according to claim 1, wherein the fluid receiving part comprises (a) a porous structure having air permeability and (b) a coating agent for coating an exterior of the porous structure such that the porous structure has airproof capability.

3. The device according to claim 1, wherein the microstructure hollow barrier is a polymer coating formed by a microstructure dipping method.

4. The device according to claim 3, wherein the polymer coating is composed of a biocompatible and biodegradable polymer.

5. The device according to claim 1, wherein the microstructure hollow barrier is a polymer cap formed by a microstructure capping method.

6. The device according to claim 5, wherein, when body fluids are collected from a subject, the polymer cap is separated from a microstructure-tip to open a hollow and form a pressure gradient when a vertical force is applied to cause the microstructure to penetrate a skin barrier of the subject.

7. The device according to claim 5, wherein the polymer cap forms an exposed-tip capping structure.

8. The device according to claim 5, wherein, when body fluids are collected from a subject, the polymer cap is separated from a microstructure-tip and does not enter a skin barrier of the subject.

9. The device according to claim 1, wherein the fluid receiving part has transparency.

10. An integrated analysis system for body fluids comprising the device for collecting body fluids according to any one of claims 1 to 9 and a device for analyzing collected fluids.

11. The integrated analysis system according to claim 10, wherein the device for analyzing collected fluids comprises a signal device for generating a fluid analysis signal.
